# EUROPEAN PATENT APPLICATION

(11) **EP 4 467 232 A1**
(43) Date of publication of application: **27.11.2024**
(21) Application number: 23175687.5
(22) Date of filing: 26.05.2023
(51) Int. Cl.: B01F 23/231, C12M 1/00

(54) **AERATION DEVICE FOR A BIOPROCESSING INSTALLATION**

(71) Applicant: Sartorius Stedim Biotech GmbH, 37079 Göttingen (DE)
(72) Inventor: Adams, Thorsten, 37079 Göttingen (DE); Kukla, Clint, 37079 Göttingen (DE)
(74) Representative: Gottschald Patentanwälte Partnerschaft mbB

(57) **Abstract**

The invention relates to a an aeration device for a bioprocessing installation (2), comprising a housing (4), wherein the housing (4) comprises a circumferential rigid body (5) extending about a geometric center axis (6) running in axial direction, wherein the housing (4) comprises at least a first aeration channel (7) and a second aeration channel (8) in its interior, the lateral extension and longitudinal extension of which are each defined by a respective recessed section in the circumferential rigid body (5), wherein the housing (4) further comprises at least a first gas inlet port (10) and a second gas inlet port (11), wherein via the first gas inlet port (10) a gas can be introduced into the first aeration channel (7) and wherein via the second gas inlet port (11) a gas can be introduced into the second aeration channel (8), and wherein the housing (4) further comprises a plurality of first gas discharge openings (12) and second gas discharge openings (13), wherein via the plurality of first gas discharge openings (12) the gas can be discharged from the first aeration channel (7) to the outside of the aeration device (1) and wherein via the plurality of second gas discharge openings (13) the gas can be discharged from the second aeration channel (8) to the outside of the aeration device (1). It is proposed that the first aeration channel (7) and the second aeration channel (8) are arranged so as to overlap one another in the axial direction at least in sections.

## Description

The present invention relates to an aeration device for a bioprocessing installation according to the general part of claim 1, to a bioprocessing installation according to claim 18 and to a method for operating a bioprocessing installation according to the general part of claim 20.

In the technological field of biotechnology, different kinds of bioprocesses are carried out to bioprocessing installations such that the respective bioprocess can be performed individually under certain conditions. Common examples for bioprocessing installations are bioreactors, in which microorganisms or mammalian cells are cultivated under certain conditions, or process mixing systems, which are used for mixing liquid biological mediums such as suspensions, solutions, emulsions or the like.

Beside other parameters, oxygen often plays a major role in bioprocesses. Thus, oxygen supply is a key factor in cellular metabolic processes, but however the sufficient supply to the cultured cells is often challenging. Also other gases, like carbon dioxide, may play an important role such that these have to be supplied besides oxygen. For supplying such gases, generally two conventional aeration methods are common in biotechnology: i) Aerating the headspace of the bioreactor and ii) direct injection of gases through aeration devices such as aeration rings or discs.

For the latter purpose, use is made not only of aeration rings or discs ("ring spargers"), which comprise gas discharge openings having a diameter larger than 0,4 mm ("macro-spargers"), for example 0,8 mm, but also of "micro-spargers", which are aeration devices with gas discharge openings with a smaller diameter, for example 0,15 mm. Both types of aeration devices have specific advantages and drawbacks. It is therefore also known to combine both types of gas discharge openings in a combined housing of an aeration device.

The known prior art (US 10,427,112 B2) that builds the basis of the invention is related to an aeration device according to claim 1. The aeration device, which is the starting point of the invention, comprises a housing, wherein the housing comprises a circumferential rigid body, which is ring-shaped with a circular geometry. The circumferential rigid body extends about a geometric center axis running in axial direction. For aeration, the housing comprises a first aeration channel and a second aeration channel in its interior, wherein the lateral extension and longitudinal extension of the aeration channels are each defined by a respective recessed section in the circumferential rigid body. Via the separately designed aeration channels several gases may be inserted or rather discharged as needed by the aeration device. Furthermore, for introducing gas into the aeration channels, the housing comprises a first and second gas inlet port, and for discharging gas from the aeration channels, the housing comprises a plurality of first and second gas discharge openings.

The known aeration device can be used for discharging different gases quite effectively. Because of the separately designed aeration channels, gas insertion can be adjusted properly, for example, by adjusting pressure inside the respective aeration channel or by choosing size and number of the gas discharge openings. Thus, the overall insertion of different gases may be properly adjusted as needed.

However, even if the known aeration device is proven for several applications, its design has challenges, too. Due to the separately designed aeration channels, which are arranged next to each other in lateral direction, the housings horizontal extension (when installed) is quite large. Thus, the known aeration device is relatively large and can only be used in bioprocessing installations with a certain width. Alternatively, its overall design has to be effortfully adapted, since the design of the aeration channels influences each other directly (for example, if the diameter of the inner aeration channel shall be increased, the diameter of the outer aeration channel has to be increased, too).

Furthermore, the handling of the aeration device, for example during installation of the aeration device inside a bioprocessing installation, is effortful, because of the unhandy overall design. Therefore, there is a need to improve the known aeration device.

The invention is based on the problem of improving the known aeration device such that an optimization regarding the forementioned challenges is reached and, in particular, an aeration device with an optimized and compact design is created.

The above-noted problem is solved by the features of the characterizing part of claim 1.

The main realization of the present invention is that the aeration channels are arranged next to each other in axial direction rather than in lateral direction. As a result, an optimized and more compact housing and thus an optimized and compact aeration device is created. Due to the more compact design, in particular in lateral direction, the aeration device may be usable in further applications, in particular in bioprocessing installations with smaller widths. Further, the handling of the aeration device is improved, for example during installation.

In detail, it is proposed that the first aeration channel and the second aeration channel are arranged so as to overlap one another in the axial direction at least in sections.

Claim 2, 3 and 4 each relate to the arrangement of the aeration channels. Here, several advantageous embodiments are specified, which may lead to a compact overall design of the aeration device, in particular regarding its lateral extension.

According to an advantageous embodiment of claim 5, membranes are utilized for separating the aeration channels from the outside of the aeration device. Membranes may improve the liability of evenly releasing gas from the aeration channels to the outside.

In claim 6, for an advantageous embodiment, it is specified that at least a part of the gas discharge openings are formed by a plurality of holes, which are comprised by the membranes. Holes may ensure uniform generation of bubbles and are easily producible inside the membranes, for example by laser drilling.

Claim 7 relates to advantageous material and geometrical design of the membranes, which showed good aeration results. Polyimide materials offer good thermal, chemical and mechanical properties. Furthermore, the materials mentioned might be easily processable by laser-drilling and/or easily sterilizable by for example gamma-irradiation.

According to an advantageous embodiment of claim 8, the gas discharge openings comprise different cross-sections and/or different diameters. This may lead to different gas release by the discharge openings and as a result to different bubble generation by the first aeration channel and the second aeration channel, wherein smaller diameters may lead to smaller bubbles and greater diameters lead to larger bubbles. Thus, the bubble generation is adjustable as needed.

Claim 9 relates to an easy and cost-effective, but equally accurate production method of the discharge openings, namely laser-drilling.

Claim 10 to 12 specify advantageous embodiments of a circumferential aeration device, wherein the circumferential rigid body comprises different sections, which delimit at least one or both aeration channels. Thus, by designing and manufacturing the circumferential rigid body, the aeration channels may be designed and manufactured herewith. As a result, an easily manufacturable aeration device is provided.

According to an advantageous embodiment of claim 13, the aeration channels each are delimited by the central radial section and the respective membrane, wherein the membrane ensures releasing gas from the respective aeration channel to the outside.

An advantageous embodiment according to claim 14 enables evenly insertion or rather discharging of gas by the aeration device.

Claim 15 refers to advantageous embodiments regarding the circumferential rigid body, wherein all embodiments provide easy manufacturing and thus cost-effective solutions.

An advantageous embodiment of claim 16 provides an easy appliable solution for attachment of the membrane to the circumferential rigid body.

An advantageous embodiment of claim 17 provides a compact design of the aeration device in its longitudinal extension.

Another teaching according to claim 18, which is of equal importance, relates to a bioprocessing installation in particular a bioreactor, comprising a container, preferably a flexible bag or a dimensionally stable vessel, in which a liquid biological medium consisting of the substances intended for a biotechnological process can be accommodated. The bioprocessing installation comprises an aeration device as proposed. The aeration device may comprise one or more of the above-mentioned features.

All explanations given with regard to the aeration device are fully applicable.

Claim 19 refers to the arrangement of the aeration device inside the container, wherein the arrangement according to the advantageous embodiments provides good aeration results.

Another teaching according to claim 20, which is of equal importance, relates to a method for operating a bioprocessing installation. The bioprocessing installation comprises a container and, inside the container, an aeration device with a housing, which comprises at least a first aeration channel and a second aeration channel in its interior, at least a first gas inlet port and a second gas inlet port and a plurality of first gas discharge openings and second gas discharge openings. Within the methods a liquid biological medium consisting of the substances intended for a biotechnological process is filled into the container so that the aeration device is surrounded by the liquid biological medium, wherein via the first gas inlet port a first gas is introduced into the first aeration channel and via the second gas inlet port a second gas, which is the same as or different from the first gas, is introduced into the second aeration channel, wherein via the plurality of first gas discharge openings the first gas is discharged from the first aeration channel to the outside of the aeration device and via the plurality of second gas discharge openings the second gas is discharged from the second aeration channel to the outside of the aeration device.

Here, it is essential that the gas flow through the first aeration channel and the gas flow through the second aeration channel overlap one another in the axial direction at least in sections.

The bioprocessing installation of the method may be the bioprocessing installation as proposed. The aeration device of the method may be the aeration device as proposed. Thus, the bioprocessing installation and/or the aeration device may comprise one or more of the above-mentioned features.

All explanations given with regard to the aeration device and the bioprocessing installation are fully applicable.

In the following, embodiments of the invention are explained with respect to the drawing. The drawing shows in
- Fig. 1: a schematic illustration of a proposed aeration device in a biopro-cessing installation in perspective view,
- Fig. 2: the aeration device according to fig. 1 in perspective view,
- Fig. 3: the aeration device according to fig. 1 in top view and in cross-sec-tional view (A-A),
- Fig. 4: the aeration device according to fig. 1 in bottom view,
- Fig. 5: a further embodiment of a proposed aeration device in top view and in cross-sectional view (A-A),
- Fig. 6: the aeration device according to fig. 5 in bottom view and
- Fig. 7: schematic illustrations of gas inlet ports in sectional partial views, wherein a) is the gas inlet port of a first aeration channel of the aera-tion device according to fig. 1, b) is the gas inlet port of a second aer-ation channel of the aeration device according to fig. 1, c) is the gas inlet port of the first aeration channel of the aeration device according to fig. 5 and d) is the gas inlet port of the second aeration channel of the aeration device according to fig. 5.

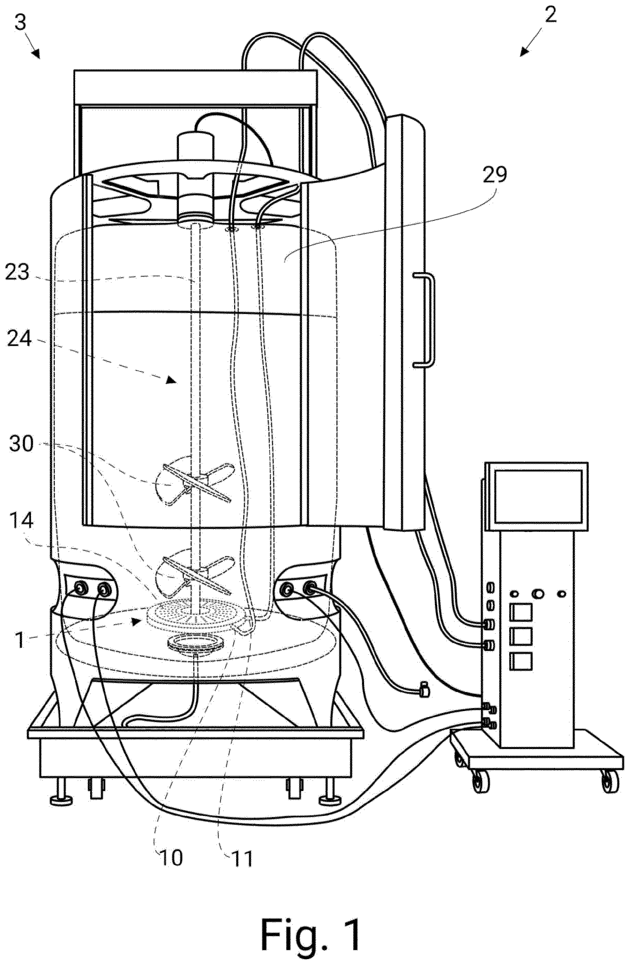

Proposed is an aeration device 1. The aeration device 1 can be used for a bioprocessing installation 2, such as a bioreactor 3 as exemplarily shown in fig. 1. Here, the aeration device 1 is installed inside the bioreactor 3 such that a fluid can be treated with gas accordingly.

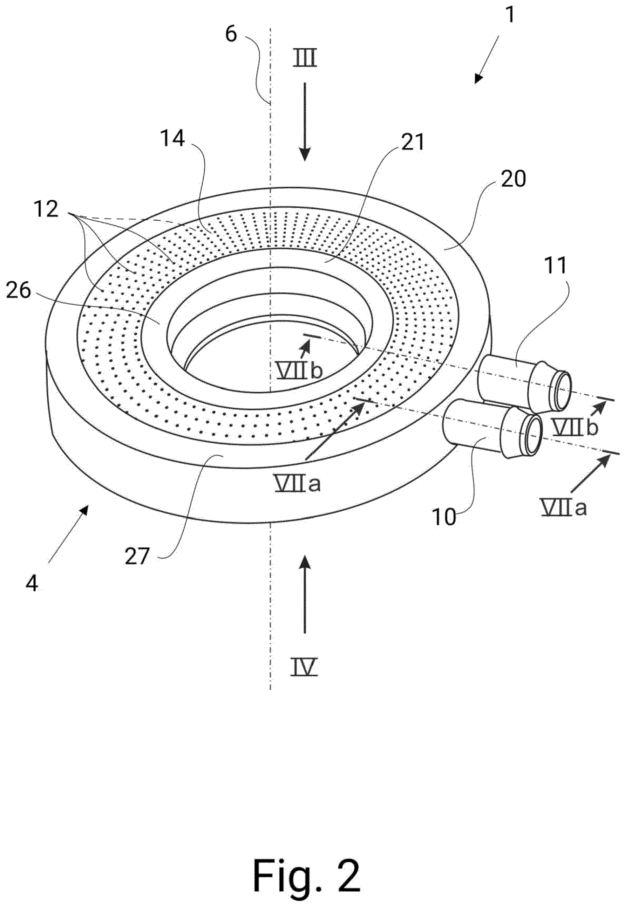

The aeration device 1, which is shown exemplarily by itself in fig. 2, comprises a housing 4. The housing 4 comprises a circumferential rigid body 5, which is preferably ring-shaped or plate-shaped, for example with a circle-shaped cross-section. The circumferential rigid body 5 extends about a geometric center axis 6, which runs in axial direction. The geometric center axis 6 is the main axis of the aeration device 1, which is orientated in vertical direction, when the aeration device 1 is installed, as exemplarily depicted in fig. 1.

The term ring-shaped includes not only a circular geometry of the circumferential rigid body 5, but also includes other shapes, for example, an elliptical geometry or polygonal geometry. Thus, around the geometric central axis 6, the inner and/or outer contour of the rigid peripheral body 5 can be circular, as shown in fig. 2, or elliptical or polygonal. Here, just as an exemplary embodiment, the housing 4, in particular the inner contour and the outer contour of the circumferential rigid body 5, surrounds the geometric center axis 6 in a primarily constant distance such that it does not intersect with the geometric center axis 6. According to another, also preferred embodiment, the inner contour may be circular and the outer contour elliptical.

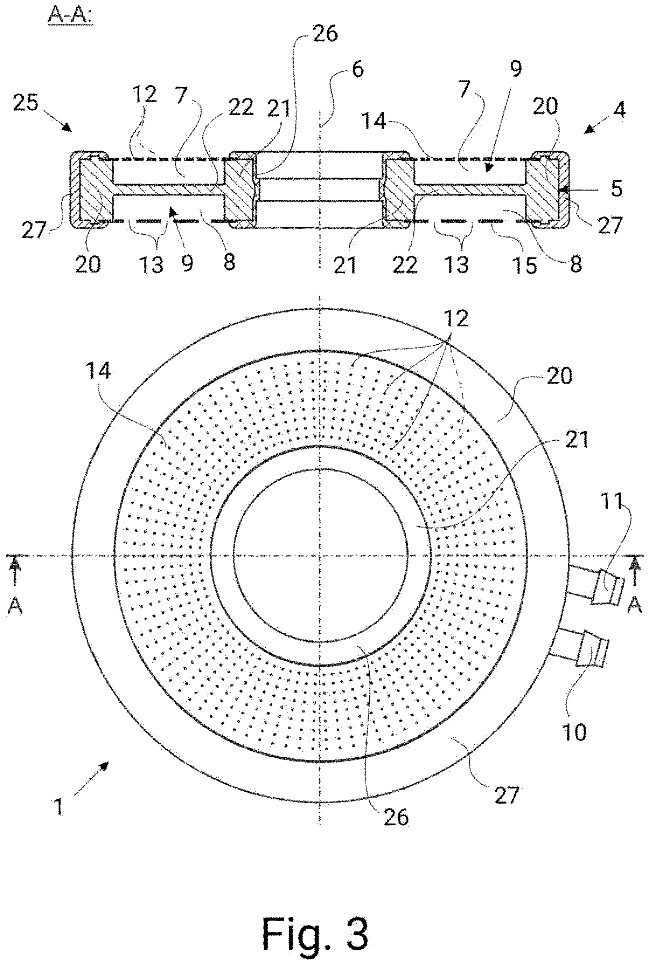

The housing 4 furthermore comprises at least a first aeration channel 7 and a second aeration channel 8. The aeration channels are arranged in the interior of the housing 4, wherein the lateral extension and longitudinal extension of the aeration channels are each defined by a respective recessed section 9 in the circumferential rigid body 5. The lateral extension runs in lateral directions, wherein the lateral directions are lateral with regard to the axial direction or rather the geometric center axis 6. The longitudinal extension runs in longitudinal directions, wherein the longitudinal directions are longitudinal with regard to the axial direction or rather the geometric center axis 6. As it can be exemplarily seen in fig. 3, the respective recessed sections 9 are preferably groove-shaped each. Here and preferably, the recessed sections 9 are designed circumferential and thus extend over 360° each. The aeration channels are, here and preferably, spatially separated.

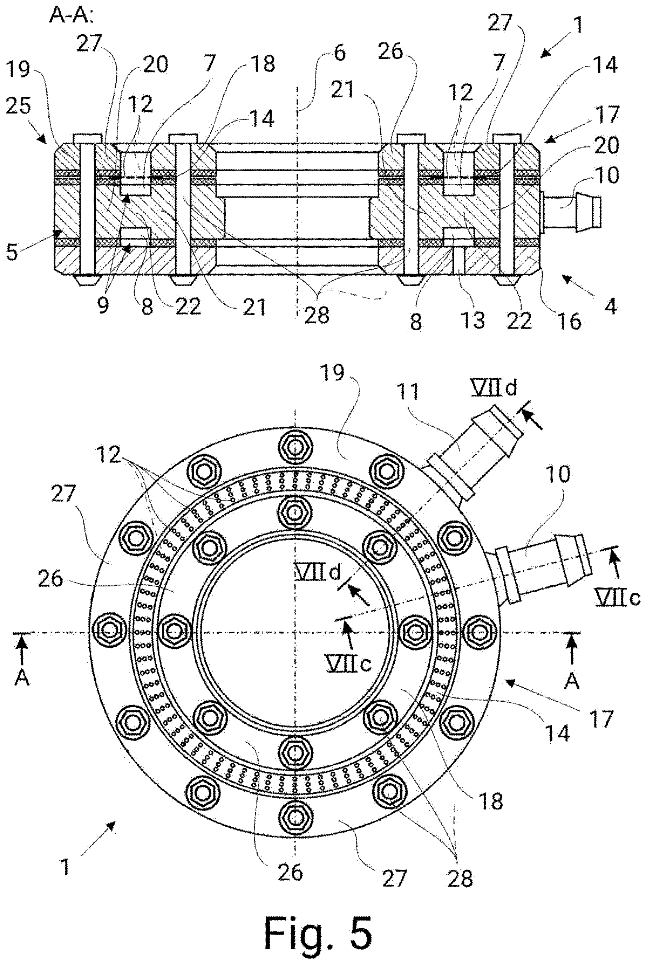

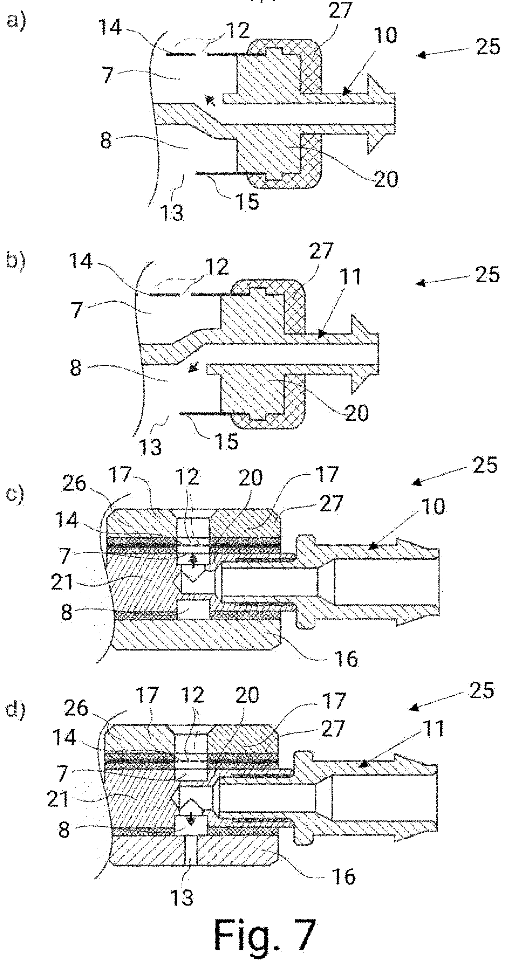

For gas entering the aeration channels, the housing 4 comprises a first gas inlet port 10 and a second gas inlet port 11. The housing 4 may comprise several first gas inlet ports 10 and/or several second gas inlet ports 11, wherein gas may enter the respective channel via the respective gas inlet ports from different direction or a larger amount of gas may enter the respective channel via the respective gas inlet ports. In general, via the first gas inlet port 10 a gas can be introduced into the first aeration channel 7 and via the second gas inlet port 11 a gas can be introduced into the second aeration channel 8. The gases, which are introduced, may be different gases, such as oxygen and carbon dioxide. In fig. 7a), 7b) and 7c), 7d), examples for preferred gas inlet ports are depicted, wherein in fig. 7a) and 7b) the sectional views are depicted with regard to lines "Vlla-Vlla" and "Vllb-Vllb" of fig. 2 and in fig. 7c) and 7d) the sectional views are depicted with regard to lines "Vllc-Vllc" and "Vlld-Vlld" of fig. 5.

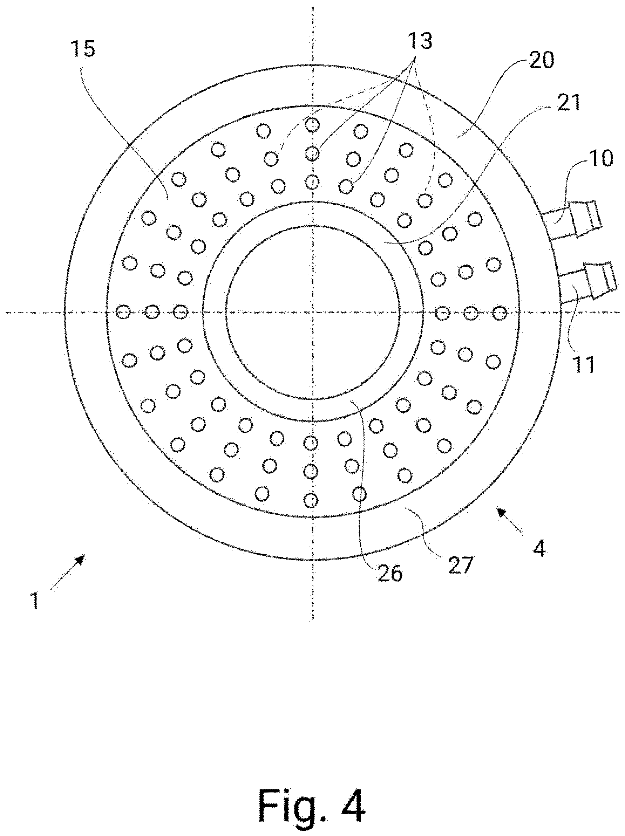

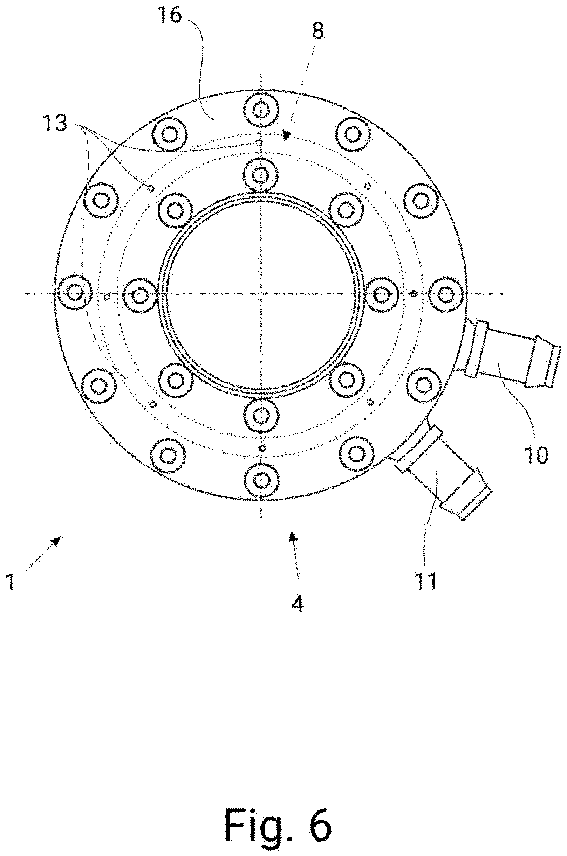

For discharging gas and, for example, inserting gas into a fluid inside the bioreactor 3 in fig. 1, the housing 4 further comprises a plurality of first gas discharge openings 12 and second gas discharge openings 13. Here, via the plurality of first gas discharge openings 12 the gas can be discharged from the first aeration channel 7 to the outside of the aeration device 1 and via the plurality of second gas discharge openings 13 the gas can be discharged from the second aeration channel 8 to the outside of the aeration device 1. Here and preferably, the first gas discharge openings 12 are arranged on the upper side of the housing 4 (fig. 3, 5) and the second gas discharge openings 13 are arranged on the lower side of the housing 4 (fig. 4, 6). Alternatively or additionally, it is also thinkable that the second gas discharge openings 13 are arranged on the side, here and preferably the lateral area, of the housing 4. In certain designs of the aeration device 1 the second gas discharge openings 13 might be arranged on the upper side of the housing 4 instead of the lower side of the housing 4 and the first gas discharge openings 12 on the lower side of the housing 4 instead of the upper side of the housing 4. It is preferred that the size of the first gas discharge openings 12 is in the range of openings of micro-spargers, in particular with a diameter smaller than 0,18 mm, preferably smaller than 0,15 mm. In an especially preferred embodiment, the size of the first gas discharge openings 12 is in the range of 40 to 60 µm. It is further preferred that the size of the second gas discharge openings 13 is in the range of openings of macro-spargers, in particular with a diameter larger than 0,2 mm, preferably larger than 0,4 mm. It is particularly preferred that the diameter of the first gas discharge openings 12 is about 0,05 mm and the size of the second gas discharge openings 13 is about 0,8 mm.

Essential is that the first aeration channel 7 and the second aeration channel 8 are arranged so as to overlap one another in the axial direction at least in sections. This does not mean that the first aeration channel 7 and the second aeration channel 8 intersect, but rather that the aeration channels overlap, if viewed along the axial direction or the geometric center axis 6 of the aeration device 1.

Thus, the first aeration channel 7 and the second aeration channel 8 are arranged next to each other with regard to the axial direction, wherein, here and preferably, the first aeration channel 7 is arranged above the second aeration channel 8.

Generally, the characteristic feature means that the first aeration channel 7 and the second aeration channel 8 are arranged in such a way that an axial projection of the first aeration channel 7 and an axial projection of the second aeration channel 8 onto a projection surface overlap one another at least in sections, wherein the projection surface is oriented orthogonally to the geometric center axis 6.

According to one embodiment it is proposed, that the first aeration channel 7 and the second aeration channel 8 run parallel to each other, at least in sections or completely. Preferably, the first aeration channel 7 and the second aeration channel 8 run parallel regarding the lateral direction, which in assembled state is the horizontal direction.

Fig. 3 shows the first aeration channel 7 and the second aeration channel 8 in a cross-sectional view. The first aeration channel 7 and the second aeration channel 8 are, here and preferably, ring-shaped. As it can be seen and preferably, the aeration channels run completely parallel to each other regarding the horizontal direction. Thus, the distance between the first aeration channel 7 and the second aeration channel 8 in axial direction is constant over the circumference.

Alternatively or additionally, the first aeration channel 7 and the second aeration channel 8 are arranged in such a way that one of the aeration channels completely overlaps the other aeration channel in the axial direction. This means that the axial projection of one of the aeration channels completely overlaps the axial projection of the other aeration channel on a projection plane.

It is furthermore preferred that the first aeration channel 7 and the second aeration channel 8 are primarily congruent in the axial direction. "Congruent" in this context means that the first aeration channel 7 and the second aeration channel 8 generally comprise the same form and the same size, but may be orientated differently. In general, the first aeration channel 7 and the second aeration channel 8 are congruent, if they can be transformed to each other by translations, rotations and/or reflections.

In fig. 5, for example, the first aeration channel 7 and the second aeration channel 8 are designed congruent. Each of the aeration channels is designed ring-shaped with a rectangular cross-section, which in particular extends over a full circle. The first channel and the second channel fully overlap in the axial direction, wherein the first channel and the second channel thus comprise the same inner and outer diameter. Regarding the congruency, the first aeration channel 7 and the second aeration channel 8 comprise the same form and the same size.

In fig. 2 and 3, the first aeration channel 7 and the second aeration channel 8 are not designed congruent. Here, the first gas inlet port 10 and the second gas inlet port 11 differ from each other regarding their direction. As a result, the first aeration channel 7 and the second aeration channel 8 cannot be transformed to each other, for example by reflection with regard to a horizontal plane and/or rotation. However, as for the embodiment according to fig. 5, here and preferably, each of the aeration channels is designed ring-shaped with a rectangular cross-section, which in particular extends over a full circle, and the first channel and the second channel fully overlap in the axial direction, wherein the first channel and the second channel thus comprise the same inner and outer diameter.

It is possible, that the first aeration channel 7 and the second aeration channel 8 are arranged so as to overlap one another in the radial direction at most partially. However, it is preferred that the first aeration channel 7 and the second aeration channel 8 are arranged so as to overlap one another in the radial direction primarily not at all. This means generally that a radial projection of the first aeration channel 7 and a radial projection of the second aeration channel 8 on a second projection surface, which second projection surface extends circumferentially around the geometric center axis 6 of the aeration device 1, are arranged so as to overlap one another at most partially, but preferably not at all. Here and preferably, the aeration channels may be arranged next to each other in axial direction, but in different horizontal planes. For example, in fig. 3 and in fig. 5, the first aeration channel 7 and the second aeration channel 8 fully overlap in axial direction, but do not overlap in radial direction at all, because the first aeration channel 7 is arranged above the second aeration channel 8.

According to another preferred embodiment it is proposed, that the first aeration channel 7 and/or the second aeration channel 8 extends concentrically to the geometric center axis 6.

As shown exemplarily in fig. 3 and 5, the aeration device 1 comprise the geometric center axis 6, which in assembled state runs in vertical direction. Here and preferably, the first aeration channel 7 and the second aeration channel 8 extend concentrically around the geometric center axis 6.

Regarding the housing 4, it is preferred, that the housing 4 comprises an upper membrane 14, wherein the upper membrane 14 axially separates the first aeration channel 7 from the outside of the aeration device 1. The upper membrane 14 might be designed corresponding to the first aeration channel 7, in particular ring-shaped, as it is exemplarily shown in fig. 3 and 5. It is possible that the upper membrane 14 separates the first aeration channel 7 from the outside such that gas is discharged by the upper membrane 14. The upper membrane 14 may function as a sealing of the first aeration channel 7, as exemplarily shown in fig. 3, or may be arranged between additional sealings, as exemplarily shown in fig. 5.

Alternatively or additionally, the housing 4 comprises a lower membrane 15, wherein the lower membrane 15 axially separates the second aeration channel 8 from the outside of the aeration device 1. The lower membrane 15 might be designed corresponding to the second aeration channel 8, in particular ring-shaped, as it is exemplarily shown in fig. 4. It is possible that the lower membrane 15 separates the second aeration channel 8 from the outside such that gas is discharged by the lower membrane 15. The lower membrane 15 may function as a sealing of the second aeration channel 8, as exemplarily shown in fig. 4, or may be arranged between additional sealings. Alternatively to a lower membrane 15, it is also possible that the second aeration channel 8 is axially separated from the outside of the aeration device 1 by a bottom plate 16, as it is exemplarily shown in fig. 6.

The term "membrane" in this context may comprise all kind of plates, sheets, foils or films with respective perforations, which acts as gas discharge openings. The maximum height of a membrane may be 1 mm at the most.

In context with the membranes, it is preferred, that the upper membrane 14 comprises a plurality of holes forming at least a part of the, preferably all, first gas discharge openings 12. This is exemplarily shown in fig. 3. Here and preferably, the upper membrane 14 delimits the first aeration channel 7 on the upper side of the channel, wherein the first discharge openings enable gas flow from the first aeration channel 7 to the outside of the aeration device 1.

Alternatively or additionally, the lower membrane 15 comprises a plurality of holes forming at least a part of the, preferably all, second gas discharge openings 13, as it is exemplarily shown in fig. 4. Here and preferably, the lower membrane 15 delimits the second aeration channel 8 on the lower side of the channel, wherein the second discharge openings enable gas flow from the second aeration channel 8 to the outside of the aeration device 1. Alternatively to the lower membrane 15, it is also possible that the bottom plate 16 comprises a plurality of holes forming at least a part of the, preferably all, second gas discharge opening 13, as it is exemplarily shown in fig. 6. Here and preferably, the bottom plate 16 delimits the second aeration channel 8 on the lower side of the channel, wherein the second discharge openings enable gas to flow through the bottom plate 16 from the second aeration channel 8 to the outside of the aeration device 1.

It is possible that the aeration device 1 comprises a pressure builder mechanism. With the pressure builder mechanism during lower flow rates of gas inside the first aeration channel 7 and/or the second aeration channel 8 still all gas discharge openings can be activated.

According to fig. 3 and preferably, the housing 4 comprises the circumferential rigid body 5, the upper membrane 14 and the lower membrane 15. Here and preferably, the upper membrane 14 and the lower membrane 15 are attached to the circumferential rigid body 5, in particular via a coating. The coating might be provided by a coating layer or alternatively by an element, which coats the circumferential rigid body 5. Here and preferably, the upper membrane 14 and the lower membrane 15 are designed self-sealed. Thus, no further sealings are required.

Preferably, the housing 4 is designed sandwich-wise, as it is exemplarily shown in fig. 5, wherein the different elements of the housing 4 are arranged one after another in axial direction. According to fig. 5 and preferably, the housing 4 comprises the circumferential rigid body 5, the upper membrane 14 and the bottom plate 16. The bottom plate 16 is preferably attached to the circumferential rigid body 5. An additional sealing may be arranged between the bottom plate 16 and the circumferential rigid body 5. Here and preferably, the housing 4 comprises a cover plate 17, which attaches the upper membrane 14 to the circumferential rigid body 5. Here and preferably, the cover plate 17 is designed two-folded, namely as an inner cover-plate 18, which is in particular ring-shaped, and an outer cover-plate 19, which is in particular ring-shaped. The upper membrane 14 is arranged between the cover plate 17 and the circumferential rigid body 5 and may be sealed with additional sealings, as exemplarily shown in fig. 5. The additional sealings may be arranged cover plate 17 sided and/or circumferential rigid body 5 sided.

According to fig. 5, a lower membrane 15 is not present, since the bottom plate 16 axially separates the second aeration channel 8 from the outside. However, it is also possible that the housing 4 comprises a lower membrane 15, which might be attached as in fig. 1 or by the bottom plate 16, which may be designed similar to the cover plate 17 in this case.

Preferably, the first and/or the second membrane is made of a material that is suited for laser drilling, does not promote cell adhesion, and is biocompatible. According to one embodiment it is proposed, that the first and/or the second membrane is made of a polyimide material, preferably Kapton. Alternatively, the upper and/or the lower membrane may at least partially be made of a plastic material like polycarbonate or of a metal material like titanium or stainless steel. It is preferred that the membrane has a thickness in a range of 10 to 300 µm, preferably in a range of 50 to 200 µm, more preferably in a range of 100 to 150 µm. It is possible that the first and/or second membrane is made of biocompatible and/or hydrophobic material.

According to one embodiment it is proposed, that the first gas discharge openings 12 each have a smaller cross-section, in particular with regard to the lateral direction of the aeration device 1, and/or a smaller diameter than the second gas discharge openings 13. As it can exemplarily be seen in fig. 3 and 4, the first gas discharge openings 12 have a smaller diameter than the second gas discharge openings 13. As a result and preferably, the gas release by the first gas discharge openings 12 differs from the gas release by the second gas discharge openings 13 such that smaller bubbles are released by the first gas discharge openings 12 and larger bubbles are released by the second gas discharge openings 13. In general, smaller bubbles comprise a larger surface-volume-ratio and thus are beneficial for gas-liquid transfer. In general, larger bubbles comprise a larger volume-surface-ratio and thus are moving up faster.

According to another embodiment it is proposed, that at least some, preferably all, of the first gas discharge openings 12 in the first membrane are laser drilled holes. It is also possible that the first gas discharge openings 12 have the shape of a circle or polygon, in particular quadrilateral. It is particularly preferred that the first gas discharge openings 12 are arranged concentrically in rings, as it can be seen in the example of fig. 3 and 5. Here and preferably, the rings have different diameters. It is preferred, if the first gas discharge openings 12 are evenly spaced, in particular in lateral and longitudinal direction, as it is shown in fig. 3 and 5. It is preferred, if the second gas discharge openings 13 are evenly spaced, in particular in lateral and/or longitudinal direction, as it is shown in fig. 4 and 6.

If the aeration device 1 comprises the second membrane, it is additionally or alternatively preferred that the second gas discharge openings 13 in the second membrane are laser drilled holes. However, if the aeration device 1 comprises the bottom plate 16, as exemplarily shown in fig. 6, the second gas discharge openings 13 may be designed as holes. In both cases, it is possible that the second gas discharge openings 13 have the shape of a circle or polygon, in particular quadrilateral. It is particularly preferred that the second gas discharge openings 13 are arranged concentrically in rings, as it can be seen in the example of fig. 4 and fig. 6.

Alternatively, the first membrane and/or the second membrane and/or the bottom plate 16 might be made out of porous material. Here the first gas discharge openings 12 and/or the second gas discharge openings 13 might be pores of the respective material.

In another preferred embodiment, the circumferential rigid body 5 comprises a completely circumferential outer radial section 20, a completely circumferential inner radial section 21 and a completely circumferential central radial section 22. The central radial section 22 extends radially from the outer radial section 20 to the inner radial section 21 and is rigidly connected to the outer radial section 20 and the inner radial section 21. Thus, the central radial section 22 connects the inner radial section 21 with the outer radial section 20 and is positioned in between. It is possible that the outer radial section 20, the inner radial section 21 and the central radial section 22 form an H-shaped cross-section of the circumferential rigid body 5, as it is exemplarily depicted in fig. 3 and 5. Here and preferred, the central radial section 22 is orientated horizontally. Here and preferred, the inner radial section 21 and the outer radial section 20 are located in the same horizontal plane, wherein the inner radial section 21 is surrounded from the outer radial section 20, in particular in a constant distance with both radial sections 20, 21 having a circular geometry, thus forming a circular ring-shape.

In another embodiment, the inner radial section 21 may be surrounded from the outer radial section 20 with a changing distance. In that case, the inner radial section 21 may have a circular shape and may be surrounded by an elliptical outer radial section 20 with both shapes having the same center. As previously indicated, however, other geometries are also conceivable for the outer contour and the inner contour of the circumferential rigid body 5, so that the inner and outer radial section 20, 21 can also be shaped differently accordingly.

Preferably, the respective radial section radially delimiting at least one of the aeration channels, in particular both aeration channels, as shown, for example, in fig. 3 and 5. Thus, the first aeration channel 7 and/or the second aeration channel 8 is or are each delimited by the inner radial section 21 on an inner side of the respective channel and/or the first aeration channel 7 and/or the second aeration channel 8 is or are each delimited by the outer radial section 20 on an outer side of the respective channel.

Preferably, the outer radial section 20 and/or the inner radial section 21 protrudes or protrude axially to one side or preferably to both sides with respect to the central radial section 22. The outer radial section 20 and/or the inner radial section 21 laterally delimits or delimit one or both of the aeration channels.

In fig. 3 and 5, for example, the outer radial section 20 and the inner radial section 21 protrude in axial direction of the aeration device 1. Both radial sections protrude, with respect to the central radial section 22, upwards and downwards such that the outer radial section 20 and the inner radial section 21 delimit the first aeration channel 7 and the second aeration channel 8 in the lateral direction.

According to another embodiment it is proposed, that the central radial section 22 axially delimits one or both of the aeration channels. As it is exemplarily depicted in fig. 3 and 5, the central radial section 22 delimits the first aeration channel 7 and the second aeration channel 8 each on one side of the respective aeration channel. It is preferred that the central radial section 22 axially separates the first aeration channel 7 from the second aeration channel 8. Hence, gases inside the aeration channels are separated inside the aeration device 1.

In a further preferred embodiment, the first aeration channel 7 is arranged axially between the upper membrane 14 and the central radial section 22. It is additionally possible that the first aeration channel 7 is arranged laterally between the inner radial section 21 and the outer radial section 20. The first aeration channel 7 is thus built by the first membrane and the radial sections, as exemplarily shown in fig. 3 and 5.

Alternatively or additionally, the second aeration channel 8 is arranged axially between the central radial section 22 and the lower membrane 15 or the bottom plate 16. It is additionally possible that the second aeration channel 8 is arranged laterally between the inner radial section 21 and the outer radial section 20. The second aeration channel 8 is thus built by the radial sections and the lower membrane 15 (fig. 3) or the bottom plate 16 (fig. 5).

According to one embodiment it is proposed, that the circumferential rigid body 5 is axially mirror-symmetrical over an angular range of at least 270°, preferably of at least 315°, more preferably of at least 337,5°. In fig. 3, for example, the circumferential rigid body 5 is axially, namely along a horizontal plane, mirror-symmetrical over an angular range of about 337,5°. Here and preferably, except the first gas inlet port 10 and the second gas inlet port 11, which differ with regard of the gas guiding into the first aeration channel 7 (fig. 7a)) or rather the second aeration channel 8 (fig. 7b)), the circumferential rigid body 5 is axially mirror-symmetrical. In fig. 5, for example, the circumferential rigid body 5 is axially, namely along a horizontal plane, mirror-symmetrical over an angular range of about 315°. Here and preferably, except the first gas inlet port 10 and the second gas inlet port 11, which differ with regard of the gas guiding into the first aeration channel 7 (fig. 7c)) or rather the second aeration channel 8 (fig. 7d)), the circumferential rigid body 5 is axially mirror-symmetrical.

It is furthermore preferred if the housing 4, in particular the circumferential rigid body 5, is ring-shaped. As shown exemplarily in fig. 1 and preferably, the ring-shaped housing 4 may enable to install the aeration device 1 to the bioprocessing installation 2, in particular via a rod 23, which may be part of a stirrer 24.

Preferably, the circumferential rigid body 5 has a round outer contour and/or a round inner contour in radial cross-section. It is possible and exemplarily depicted in fig. 3 and 5 that the round outer counter and/or the round inner contour build a circular path each.

According to a further embodiment it is proposed, that the circumferential rigid body 5 is in one piece. Here, the inner radial section 21, the central radial section 22 and the outer radial section 20 are designed rigidly and continuously, namely in one piece, which might be produced by injection molding. It is preferred that the circumferential rigid body 5 is an injection-molded part. Furthermore, it is preferred, that the circumferential rigid body 5 is made of a plastic material, preferably polycarbonate.

Regarding the design of the circumferential rigid body 5, it is possible that the circumferential rigid body 5 is provided with a coating. The coating might be a coating, which is of the same or a harder or softer material in relation to the material of the circumferential rigid body 5. The coating might be a plastic material. The coating might be biocompatible. It is in particular possible that the outer radial section 20 and/or the inner radial section 21 of the circumferential rigid body 5 are provided with a coating. A coating might prove a safer handling, in particular when transporting or installing the aeration device 1 to a bioprocessing installation 2, like a bioreactor 3. The coating might be applied in particular in form of a coating layer.

Preferably, the outer radial section 20 and the inner radial section 21 are provided at least partially with a coating. Here and preferred, the coating overlaps an edge region of the upper membrane 14 and the lower membrane 15 in particular such that the coating fixes the membranes to the circumferential rigid body 5. It is also possible that only a single membrane, in particular the upper membrane 14, is overlapped and fixed by the coating.

It is also possible that the lower membrane 15 and/or the upper membrane 14 is or are attached to the circumferential rigid body 5, in particular to opposite sites of the circumferential rigid body 5. The lower membrane 15 and/or the upper membrane 14 may be attached by form closure, friction closure and/or adhesive bond. For example, the respective membrane might be glued and/or clamped and/or welded to the circumferential rigid body 5.

It is preferred that the aeration device 1 comprises a membrane attachment arrangement 25, which attaches the lower membrane 15 and/or the upper membrane 14 to the circumferential rigid body 5. Further preferably, the membrane attachment arrangement 25 comprises an inner attachment element 26 for attaching the lower membrane 15 and/or the upper membrane 14 to the inner radial section 21, as it is exemplarily shown in fig. 3. Here and preferably, the attachment arrangement comprises an outer attachment element 27 for attaching the lower membrane 15 and/or the upper membrane 14 to the outer radial section 20, as it is also exemplarily shown in fig. 3. It is particularly preferred that the inner attachment element 26 and/or the outer attachment element 27 is or are arranged to the circumferential rigid body 5 by an overmolding process, like it is exemplarily shown in fig. 3. Here and preferably, the inner attachment element 26 and the outer attachment element 27 are each ring-shaped. Here and preferred, the inner attachment element 26 and the outer attachment element 27 partly overlaps an edge region of the upper membrane 14 and the lower membrane 15. As depicted in fig. 5 and preferably, the inner cover-plate 18 might be designed as the inner attachment element 26 and/or the outer cover-plate 19 might be designed as the outer attachment element 27. Here and preferably, the inner cover-plate 18 and the outer cover-plate 19 attach the upper membrane 14 to the circumferential rigid body 5.

In an alternative embodiment and as indicated in fig. 5, elongated fasteners 28, e.g. bolt type fasteners or the like, here threaded bolts with corresponding nuts, may be used to fasten the upper membrane 14 between the respective cover-plate 18, 19 and the rigid body 5 as well as the rigid body 5 to the bottom plate 19.

A further preferred embodiment intends that the middle axis of the first gas inlet port 10 and the middle axis of the second gas inlet port 11 are arranged in the same radial or axial plane. The first gas inlet port 10 and the second gas inlet port 11 may be designed cylindrically. Therefore, the first gas inlet port 10 and the second gas inlet port 11 may comprise a respective middle axis each. According to fig. 3 and 5, the middle axes of the gas inlet ports are arranged in the same radial plane, namely the same horizontal plane. It is generally preferred that the middle axes of the gas inlet ports primarily intersect with the geometric center axis 6 of the aeration device 1, in particular at the same point, as it is exemplarily shown in fig. 5. However, it is alternatively also possible that one of the middle axes does not intersect with the geometric center axis 6 of the aeration device 1 at all, as it is exemplarily depicted in fig. 3.

Alternatively to a cylindrical design, the respective gas inlet ports 10, 11 may be designed such that the part of the gas inlet port 10, 11 where the respective gas is introduced, is inclined with respect to the middle axis. Preferably, the angle between the middle axis and the part of the gas inlet port 10, 11 where the gas is introduced, is 45°.

Additional fixing bars (not depicted) may be used to additionally secure the upper membrane 14 and/or, if present, also the lower membrane 15.

Another teaching which is of equal importance relates to a bioprocessing installation 2. The bioprocessing installation 2 may be a bioreactor 3. The bioprocessing installation 2 comprises a container 29, in which a liquid biological medium consisting of the substances intended for a biotechnological process can be accommodated, and an aeration device 1, as proposed with one or more of the provided features. The container 29 may be a flexible bag, which might be attached to a rack, or a dimensionally stable vessel. The bioprocessing installation 2 particularly comprises a stirrer 24.

Preferably and as exemplarily depicted in fig. 1, the stirrer 24 and the aeration device 1 are coaxially arranged inside of the container 29. It is possible that the stirrer 24 comprises a rod 23. The aeration device 1 may be attached to the rod 23. Alternatively, the aeration device 1 is attached to the container 29 by an attachment element or attachment elements. For example the aeration device 1 might be attached by hanging, in particular via supply pipes, or by mounting, in particular via a mount attached to the bottom of the container 29. Furthermore, the stirrer 24 may comprise at least one, preferably several, stirring elements 30 for stirring the fluid, which is contained in the container 29. In axial direction, the aeration device 1 is preferably arranged below the stirring element 30 or stirring elements 30, in particular, such that rising gas bubbles are distributed by the stirring element 30 or stirring elements 30.

It is preferred that the aeration device 1 is attached inside the container 29 such that the aeration device 1 is contactless with regard to the container 29 itself, in particular contactless with regard to the bottom of the container 29, as exemplarily shown in fig. 1. Due to the space between the bottom of the container 29 and the aeration device 1, gas from the second gas channel, namely second gas, can be discharged properly. In fig. 1, for example, the aeration device 1 is rotatable attached to the rod 23 of the stirrer 24 such that the aeration device 1 is distanced to the bottom of the container 29. It is alternatively also possible and preferred that the bioprocessing installation 2 comprises an attachment element for attaching the aeration device 1 into the container 29. The attachment element might be designed to hang, for example designed as supply pipes, and/or mount, for example designed as a mount, the aeration device 1.

According to another preferred embodiment it is proposed, that the aeration device 1 is arranged such that the upper membrane 14 covering the first aeration channel 7 towards the axial top of the container 29 and that the lower membrane 15 covering the second aeration channel 8 faces towards the axial bottom of the container 29. The axial top of the container 29 and the axial bottom of the container 29 are the upper or rather lower end of the container 29 with regard to the vertical direction when the container 29 is installed according to its intended use. Here and preferably, gas from the first aeration channel 7, namely first gas, and gas from the second aeration channel 8, namely second gas, are discharged in opposite directions. Gas from the first aeration channel 7 is thus be discharged in axial direction upwards and gas from the second aeration channel 8 is thus be preferably discharged in axial direction downwards.

Alternatively, the aeration device 1 is arranged such that the upper membrane 14 covering the first aeration channel 7 faces towards the axial bottom of the container 30 and that the lower membrane 15 covering the second aeration channel 8 faces towards the axial top of the container. In this case, gas from the first aeration channel 7 is thus be discharged in axial direction downwards and gas from the second aeration channel 8 is thus be preferably discharged in axial direction upwards.

Alternatively, it is also possible that gas from the second aeration channel 8 is discharged sideways. Here and preferably, the second gas discharging openings 13 are arranged accordingly sideways of the aeration device 1.

Additionally or alternatively, the aeration device 1 is arranged such that the gas is directed through the plurality of first gas discharge openings 12 towards the axial top of the container 29 and the gas is directed through the plurality of second gas discharge openings 13 towards the axial bottom (fig. 1) of the container 29 (fig. 1). However, it is also possible that gas is directed through the plurality of second gas discharge openings 13 towards the sides of the container 29.

Alternatively, the aeration device 1 may be arranged such that the gas is directed through the first gas discharge openings 12 towards the axial bottom of the container 29 and the gas is directed through the second gas discharge openings 13 towards the axial top of the container 29. In this case, gas from the first aeration channel 7 is thus be discharged in axial direction downwards and gas from the second aeration channel 8 is thus be discharged in axial direction upwards towards the bottom of the container 29.

Another teaching which is of equal importance relates to a method for operating a bioprocessing installation 2, in particular as proposed with one or more of the provided features, wherein the bioprocessing installation 2 comprises a container 29 and, inside the container 29, an aeration device 1, in particular as proposed with one or more of the provided features, with a housing 4 comprising at least a first aeration channel 7 and a second aeration channel 8 in its interior, at least a first gas inlet port 10 and a second gas inlet port 11 and a plurality of first gas discharge openings 12 and second gas discharge openings 13. The method comprises that a liquid biological medium consisting of the substances intended for a biotechnological process is filled into the container 29 so that the aeration device 1 is surrounded by the liquid biological medium, and via the first gas inlet port 10 a first gas is introduced into the first aeration channel 7 and via the second gas inlet port 11 a second gas, which is the same as or different from the first gas, is introduced into the second aeration channel 8, and via the plurality of first gas discharge openings 12 the first gas is discharged from the first aeration channel 7 to the outside of the aeration device 1 and via the plurality of second gas discharge openings 13 the second gas is discharged from the second aeration channel 8 to the outside of the aeration device 1.

Here, it is essential, that the gas flow through the first aeration channel 7 and the gas flow through the second aeration channel 8 overlap one another in the axial direction at least in sections.

This means that the first aeration channel 7 is flowed through by the first gas and the second aeration channel 8 is flowed through by the second gas in such a way that an axial projection of the gas flow in the first aeration channel 7 and an axial projection of the gas flow in the second aeration channel 8 onto a first projection surface, which is oriented orthogonally to the geometric center axis 6, overlap one another at least in sections.

## Claims

1. An aeration device for a bioprocessing installation (2), in particular a bioreactor (3), comprising a housing (4),
wherein the housing (4) comprises a circumferential rigid body (5), in particular a ring-shaped or plate-shaped rigid body, extending about a geometric center axis (6) running in axial direction,
wherein the housing (4) comprises at least a first aeration channel (7) and a second aeration channel (8) in its interior, the lateral extension and longitudinal extension of which are each defined by a respective recessed, in particular groove-shaped, section in the circumferential rigid body (5),
wherein the housing (4) further comprises at least a first gas inlet port (10) and a second gas inlet port (11), wherein via the first gas inlet port (10) a gas can be introduced into the first aeration channel (7) and wherein via the second gas inlet port (11) a gas can be introduced into the second aeration channel (8), and
wherein the housing (4) further comprises a plurality of first gas discharge openings (12) and second gas discharge openings (13), wherein via the plurality of first gas discharge openings (12) the gas can be discharged from the first aeration channel (7) to the outside of the aeration device (1) and wherein via the plurality of second gas discharge openings (13) the gas can be discharged from the second aeration channel (8) to the outside of the aeration device (1),
**characterized in**
**that** the first aeration channel (7) and the second aeration channel (8) are arranged so as to overlap one another in the axial direction at least in sections.

2. An aeration device according to claim 1, **characterized in that** the first aeration channel (7) and the second aeration channel (8) run parallel to each other, at least in sections or completely, and/or are arranged in such a way that one aeration channel completely overlaps the other aeration channel in the axial direction, preferably, that the first aeration channel (7) and the second aeration channel (8) are congruent in the axial direction.

3. An aeration device according to claim 1 or 2, **characterized in that** the first aeration channel (7) and the second aeration channel (8) are arranged so as to overlap one another in the radial direction at most partially, but preferably not at all.

4. An aeration device according to one of the preceding claims, **characterized in that** the first aeration channel (7) and/or the second aeration channel (8) extends concentrically to the geometric center axis (6).

5. An aeration device according to one of the preceding claims, **characterized in that** the housing (4) comprises an upper membrane (14), the upper membrane (14) axially separating the first aeration channel (7) from the outside of the aeration device (1), and/or, a lower membrane (15) or a bottom plate (16) of the housing (4), wherein the lower membrane (15) or the bottom plate (16) axially separating the second aeration channel (8) from the outside of the aeration device (1).

6. An aeration device according to claim 5, **characterized in that** the upper membrane (14) comprises a plurality of holes forming at least part of the, preferably all, first gas discharge openings (12) and/or that the lower membrane (15) comprises a plurality of holes forming at least part of the, preferably all, second gas discharge openings (13).

7. An aeration device according to claim 5 or 6, **characterized in** the membrane is made of a polyimide material, preferably Kapton, and/or has a thickness in a range of 1 to 300 µm, preferably in a range of 50 to 200 µm, more preferably in a range of 100 to 150 µm.

8. An aeration device according to one of the preceding claims, **characterized in that** the first gas discharge openings (12) have a smaller cross-section and/or a smaller diameter than the second gas discharge openings (13).

9. An aeration device according to one of the preceding claims, **characterized in that** at least some, preferably all, of the first gas discharge openings (12) and/or the second gas discharge openings (13), in particular in the membrane, are laser drilled holes, and/or, have the shape of a circle or polygon, in particular quadrilateral, and/or, are arranged concentrically in rings.

10. An aeration device according to one of the preceding claims, **characterized in that** the circumferential rigid body (5) comprises a completely circumferential outer radial section (20), a completely circumferential inner radial section (21) and a completely circumferential central radial section (22) which extends radially from the outer radial section (20) to the inner radial section (21) and is rigidly connected to the outer radial section (20) and the inner radial section (21), the respective radial section radially delimiting at least one of the aeration channels, in particular both aeration channels.

11. An aeration device according to claim 10, **characterized in that** the outer radial section (20) and/or the inner radial section (21) protrudes axially to at least one side, preferably to both sides, with respect to the central radial section (22), and laterally delimits at least one of the aeration channels, in particular both aeration channels.

12. An aeration device according to claim 10 or 11, **characterized in that** the central radial section (22) axially delimits at least one of the aeration channels, in particular both aeration channels, preferably **in that** the central radial section (22) axially separates the first aeration channel (7) from the second aeration channel (8).

13. An aeration device according to one of claims 10 to 12, **characterized in that** the first aeration channel (7) is arranged axially between the upper membrane (14) and the central radial section (22) and/or that the second aeration channel (8) is arranged axially between the lower membrane (15) and the central radial section (22).

14. An aeration device according to one of the preceding claims, **characterized in that** the circumferential rigid body (5) is axially mirror-symmetrical over an angular range of at least 270°, preferably of at least 315°, more preferably of at least 337,5°, and/or, preferably when ring-shaped, has a round outer contour and/or round inner contour in radial cross-section.

15. An aeration device according to one of the preceding claims, **characterized in that** the circumferential rigid body (5) is in one piece and/or is an injection-molded part, and/or, is made of a plastic material, preferably polycarbonate.

16. An aeration device according to one of claims 13 to 15, **characterized in that** the lower membrane (15) and/or the upper membrane (14) is or are attached to the circumferential rigid body (5), in particular to opposite sites of the circumferential rigid body (5), preferably that the aeration device (1) comprises a membrane attachment arrangement (25), which attaches the lower membrane (15) and/or the upper membrane (14) to the circumferential rigid body (5), further preferably that the membrane attachment arrangement (25) comprises an inner attachment element (26) for attaching the lower membrane (15) and/or the upper membrane (14) to the inner radial section (21) and an outer attachment element (27) for attaching the lower membrane (15) and/or the upper membrane (14) to the outer radial section (20).

17. An aeration device according to one of the preceding claims, **characterized in that** the middle axis of the first gas inlet port (10) and the middle axis of the second gas inlet port (11) are arranged in the same radial or axial plane.

18. A bioprocessing installation in particular a bioreactor (3), comprising a container (29), preferably a flexible bag or a dimensionally stable vessel, in which a liquid biological medium consisting of the substances intended for a biotechnological process can be accommodated, further comprising an aeration device (1) according to one of the preceding claims and in particular a stirrer (24).

19. A bioprocessing installation according to claim 18, **characterized in that** the aeration device (1) is arranged such that the upper membrane (14) covers the first aeration channel (7) towards the axial top of the container (29) and that the lower membrane (15) or the bottom plate (16) covers the second aeration channel (8) towards the axial bottom of the container (29), and/or,
that the aeration device (1) is arranged such that the gas is directed through the plurality of first gas discharge openings (12) towards the axial top of the container (29) and the gas is directed through the plurality of second gas discharge openings (13) towards the axial bottom, towards the sides or towards the axial top of the container (29).

20. A method for operating a bioprocessing installation, in particular according to claim 18 or 19, comprising a container (29) and, inside the container (29), an aeration device (1), in particular according to one of claims 1 to 17, with a housing (4) comprising at least a first aeration channel (7) and a second aeration channel (8) in its interior, at least a first gas inlet port (10) and a second gas inlet port (11) and a plurality of first gas discharge openings (12) and second gas discharge openings (13),
wherein a liquid biological medium consisting of the substances intended for a biotechnological process is filled into the container (29) so that the aeration device (1) is surrounded by the liquid biological medium,
wherein via the first gas inlet port (10) a first gas is introduced into the first aeration channel (7) and via the second gas inlet port (11) a second gas, which is the same as or different from the first gas, is introduced into the second aeration channel (8),
wherein via the plurality of first gas discharge openings (12) the first gas is discharged from the first aeration channel (7) to the outside of the aeration device (1) and via the plurality of second gas discharge openings (13) the second gas is discharged from the second aeration channel (8) to the outside of the aeration device (1), **characterized in that**
the gas flow through the first aeration channel (7) and the gas flow through the second aeration channel (8) overlap one another in the axial direction at least in sections.
